(19) **Europäisches Patentamt European Patent Office Office européen des brevets**

(11) **EP 3 601 946 B1**

(12) **FASCICULE DE BREVET EUROPEEN**

(45) Date de publication et mention de la délivrance du brevet:
**04.05.2022 Bulletin 2022/18**

(21) Numéro de dépôt: **18712527.3**

(22) Date de dépôt: **12.03.2018**

(51) Classification Internationale des Brevets (IPC):
**G01B 9/02** *(2022.01)* **G01B 11/06** *(2006.01)*
**A61B 5/00** *(2006.01)* **G01N 21/47** *(2006.01)*

(52) Classification Coopérative des Brevets (CPC):
**G01B 9/0209; G01B 9/02044; G01B 9/02057; G01B 9/02064; G01B 9/02065; G01B 9/02078; G01B 9/02085; G01B 11/0625; G01B 11/0675;** A61B 5/0066; G01B 2210/56; G01B 2290/35; G01N 21/4795

(86) Numéro de dépôt international:
**PCT/EP2018/056056**

(87) Numéro de publication internationale:
**WO 2018/172119 (27.09.2018 Gazette 2018/39)**

(54) **DISPOSITIF ET PROCEDE DE REFLECTOMETRIE A FAIBLE COHERENCE A DETECTION TEMPS-FREQUENCE**

VERFAHREN UND VORRICHTUNG ZUR KURZKOHÄRENTEN REFLEKTOMETRIE UNTER VERWENDUNG VON ZEIT-FREQUENZ-ERFASSUNG

LOW-COHERENCE REFLECTOMETRY METHOD AND DEVICE EMPLOYING TIME-FREQUENCY DETECTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.03.2017 FR 1752314**

(43) Date de publication de la demande:
**05.02.2020 Bulletin 2020/06**

(73) Titulaire: **Fogale Nanotech 30900 Nîmes (FR)**

(72) Inventeurs:
• **COURTEVILLE, Alain 30111 Congenies (FR)**
• **NEEL, Christian 30900 Nîmes (FR)**
• **GODAVARTHI, Charankumar 30900 Nîmes (FR)**

(74) Mandataire: **IPAZ Parc Les Algorithmes, Bâtiment Platon CS 70003 Saint-Aubin 91192 Gif-sur-Yvette Cedex (FR)**

(56) Documents cités:
WO-A1-2017/108400    US-A1- 2009 244 547
US-A1- 2011 181 889    US-A1- 2012 257 207
US-A1- 2012 320 380    US-A1- 2016 038 023

## Description

### Domaine technique

La présente invention concerne un dispositif et un procédé de réflectométrie à faible cohérence à détection temps-fréquence.

Le domaine de l'invention est plus particulièrement mais de manière non limitative celui des dispositifs optiques de mesures de structures d'objets, de distances et d'épaisseurs.

### Etat de la technique antérieure

On connaît différentes techniques optiques basées sur l'interférométrie à faible cohérence pour mesurer des distances ou des épaisseurs de matériaux, ou des structures internes. Ces techniques sont en général connues sous le nom d'OCT (« Optical Coherence Tomography » en Anglais) ou OCR (« Optical Coherence Reflectometry » en Anglais).

On connaît des techniques basées sur une détection dans le domaine temporel (TD-OCR, « Time-Domain OCR » en Anglais). Une ligne à retard introduit un retard variable entre un faisceau de référence et un faisceau de mesure issu de l'objet à mesurer. Les deux faisceaux interfèrent sur un détecteur d'intensité et produisent des interférogrammes lorsque le retard entre le faisceau de mesure réfléchi dans l'objet et le faisceau de référence est inférieur à la longueur de cohérence de la source optique. La position des interférogrammes dans la course de la ligne à retard est représentative de la position des interfaces.

Ces techniques présentent l'avantage que l'étendue de mesure n'est limitée vers les grandes distances que par la course de la ligne à retard, et peut facilement atteindre plusieurs dizaines de centimètres.

Elles ont par contre l'inconvénient que les cadences de mesures sont limitées par la vitesse de déplacement de la ligne à retard et qu'elles sont de ce fait sensibles aux vibrations. En outre elles nécessitent l'utilisation de sources optiques à large spectre émettant en continu, du fait des besoins en échantillonnage temporel à haute cadence du signal d'interférence. En effet, les sources impulsionnelles ou à balayage de fréquence ont des fréquences de répétition trop lentes et incompatibles avec les fréquences d'échantillonnage temporelle requises. Or les sources continues disponibles en pratique sont limitées en termes de largeur spectrale, ce qui limite les épaisseurs minimales mesurables.

On connaît également des techniques basées sur une détection dans le domaine fréquentiel ou spectral. La lumière issue de réflexions sur des interfaces d'un objet à mesurer est dirigée vers un détecteur qui permet de mesurer l'intensité des signaux d'interférences générés à une pluralité de longueurs d'ondes. Un faisceau de référence est parfois également utilisé pour définir une référence de position.

On connaît notamment des techniques basées sur une source lumineuse à large spectre et une détection de type spectrale, avec un détecteur de type spectromètre qui permet de mesurer l'intensité lumineuse reçue en fonction de la longueur d'onde optique (FD-OCR, « Frequency Domain OCR » en Anglais)

On connaît également des techniques basées sur une source laser accordable ou à balayage en longueur d'onde et une détection en intensité (SS-OCR, « Swept Source OCR » en Anglais).

Dans les deux cas, on obtient en sortie du détecteur une mesure de la densité spectrale de puissance du spectre de la lumière reçue, qui correspond au spectre de la source modulé en fonction des contributions et des différences de trajets optiques des différentes réflexions qui y contribuent. L'analyse de ce spectre, par exemple par transformée de Fourier, permet de mesurer les épaisseurs entre les interfaces de l'objet, et/ou localiser ces interfaces.

Les techniques avec une détection dans le domaine spectral ont l'avantage de permettre une meilleure sensibilité de mesure que les techniques avec une détection dans le domaine temporel, et des cadences de mesures très rapides limitées uniquement par les cadences de mesure des détecteurs. Elles ont en outre l'avantage de permettre l'utilisation de sources impulsionnelles ou à balayage qui permettent d'obtenir des largeurs spectrales plus importantes que les sources continues, et qui permettent ainsi de mesurer des épaisseurs fines.

Elles présentent toutefois l'inconvénient que l'interprétation des mesures peut être difficiles pour des objets de structure complexe, en particulier du fait que les mesures de distances déduites de la densité spectrale de puissance sont des valeurs non-signées.

En outre, les techniques spectrales ne permettent qu'une étendue de mesure limitée vers les grandes distances, avec en particulier un rapport signal à bruit qui se dégrade vers les grandes distances à cause d'effets d'échantillonnage du spectre et de la longueur de cohérence de la lumière détectée.

Ainsi, par exemple, une source de spectre carré centrée sur une longueur d'onde centrale $\lambda_0$ de largeur $\Delta\lambda$ a une longueur de cohérence de l'ordre de :

$$l_c = (1/2)\ (\lambda_0{}^2/\Delta\lambda)$$

Cette longueur de cohérence définit l'épaisseur optique minimale $L_{min}$ mesurable.

Dans un dispositif à détection spectrale avec un spectromètre permettant d'acquérir $N$ points de mesure dans la gamme de longueurs d'onde $\Delta\lambda$ (par exemple sur $N$ pixels d'un capteur linéaire), ou dans un dispositif mettant en œuvre une source laser accordable permettant de générer $N$ longueurs d'ondes différentes dans la gamme de longueurs d'onde $\Delta\lambda$, l'épaisseur optique maximale mesurable $L_{max}$ est :

$$L_{max} = (N/4)(\lambda_0{}^2/\Delta\lambda)$$

Ainsi, l'étendue de mesure d'un système spectral correspond à :

$$L_{max}/L_{min} = L_{max}/l_c = N/2$$

Elle est donc directement limitée par le nombre de points de mesure du spectromètre (ou, avec une source accordable, par le nombre de longueur d'ondes distinctes qui peuvent être générées).

En pratique, les spectromètres comprennent des capteurs avec quelques centaines de points, et les étendues de mesure des systèmes spectraux sont en général de l'ordre d'une centaine de fois la longueur de cohérence de la source.

Les techniques de type TD-OCR ou FD-OCR peuvent être mises en œuvre sous différentes formes. On connaît par exemple le document US 7,426,036 qui décrit un dispositif de type FD-OCR. Ce dispositif met en œuvre une configuration d'interféromètre de type Fizeau ou en chemin commun qui permet de générer un faisceau de référence optique dans le même bras de l'interféromètre que le faisceau de mesure. Le faisceau de référence est utilisé pour générer une référence optique à partir de laquelle les distances des interfaces de l'objet à mesurer sont déterminées. Il comprend également une ligne à retard qui permet de positionner de manière statique la position de la référence optique.

Parmi les sources existantes utilisables en interférométrie à faible cohérence, et en particulier qui peuvent être efficacement couplées dans des fibres optiques monomodes, on connaît des sources optiques de type ASE (« Amplified spontaneous émission » en Anglais) ou de type diodes superluminescentes (SDL). Ces sources permettent une émission de lumière en continu. Elles peuvent donc être mises en œuvre avec des techniques de type TD-OCR et FD-OCR. Elles présentent toutefois l'inconvénient d'avoir une largeur spectrale limitée, par exemple inférieure à 100 nm autour de 1310 nm, ce qui correspond à une épaisseur optique (ou dans l'air) minimum mesurable de l'ordre de 10 μm.

On connaît également des sources optiques qui permettent de générer des spectres plus larges, et qui ont donc des longueurs de cohérences plus courtes. Ces sources permettent d'atteindre des épaisseurs minimales mesurables inférieures (par exemple de l'ordre de 5 μm ou moins). Toutefois, elles sont soit impulsionnelles (tel que les sources laser supercontinuum qui génèrent un spectre large en injectant des impulsions de lumière issues d'un laser dans un milieu fortement non-linéaire tel qu'une fibre à cristal photonique) soit à balayage (laser accordables), avec des fréquences de répétitions ou de balayage souvent de l'ordre de quelques kilohertz. Elles sont donc incompatibles avec besoins en fréquence d'acquisition de signal des détections de type TD-OCR, et sont utilisées uniquement avec des techniques de détection spectrale.

Or certaines applications industrielles, notamment dans l'industrie du semiconducteur, nécessitent de pouvoir mesurer à la fois des épaisseurs de quelques microns et des distances optiques de l'ordre de quelques millimètres, ce qui requiert une étendue de mesure qui n'est pas atteignable avec les systèmes connus.

US 2012/257207 A1 divulgue un dispositif interféromètre à faible cohérence pour mesurer simultanément l'épaisseur de couches individuelles d'un wafer, la profondeur de caractéristiques gravées sur un wafer, ainsi que le profil tridimensionnel d'un wafer.

La présente invention a pour but de proposer un dispositif et un procédé de mesure de distances et d'épaisseurs qui résolve les inconvénients de l'art antérieur.

La présente invention a également pour but de proposer un tel dispositif avec une gamme de mesure étendue.

La présente invention a également pour but de proposer un tel dispositif qui permette de mesurer des épaisseurs ou des distances très petites.

La présente invention a également pour but de proposer un tel dispositif qui permette des mesures de grande précision.

**Exposé de l'invention**

Cet objectif est atteint avec un dispositif interféromètre à faible cohérence pour déterminer des informations sur la structure et/ou la localisation d'interfaces d'un objet tel que défini par la revendication 1. Il comprend :

- une source de lumière polychromatique ;
- un système optique pour générer un faisceau optique de mesure réfléchi par ledit objet, et un faisceau optique de référence ;
- une ligne à retard pour introduire un délai optique variable entre le faisceau optique de mesure et le faisceau optique de référence ;
- une détection optique pour combiner le faisceau optique de mesure et le faisceau optique de référence, et produire un signal spectral représentatif d'une densité spectrale de puissance optique du signal d'interférences résultant;
  caractérisé en ce qu'il comprend en outre un module de contrôle et de traitement agencé pour :

- acquérir une pluralité de signaux spectraux pour une pluralité de délais optiques ;
- déterminer pour chaque signal spectral des informations de retard optique entre faisceaux interférents dans une gamme de mesure dite spectrale ;
- analyser l'évolution desdits retards optique en fonction du délai optique et affecter le ou les retards optiques déterminés à partir des différents signaux spectraux à une ou des courbes dites d'interface, correspondant à des droites de pente unitaire positive ou négative, ou de pente nulle, en fonction du délai optique respectif de l'acquisition desdits signaux spectraux; et

- en déduire des informations sur la structure et/ou la localisation d'interfaces de l'objet en utilisant la ou les courbes d'interface.

La gamme de mesure spectrale peut bien entendu être exprimée en temps (retard optique) ou en distances optiques (différences de marche des faisceaux). On rappelle que les distances optiques, les trajets optiques et les épaisseurs optiques correspondent à des distances géométriques multipliées par l'indice de réfraction des milieux traversés.

De même le délai optique introduit par la ligne à retard peut être exprimé en temps ou en distance optique.

Par ailleurs, les termes « retard optique » ou « délai optique » expriment des différences de temps de parcours ou des décalages temporels entre faisceaux issus de la même source.

Une courbe d'interface peut par exemple représenter l'évolution d'un retard optique lié à des interférences entre le faisceau optique de référence et le faisceau optique de mesure tel que réfléchi par une interface particulière de l'objet en fonction du délai optique.

Suivant des modes de réalisation, le dispositif de l'invention peut comprendre une source de lumière émettant une lumière polychromatique sous forme d'impulsions et un détecteur optique de type spectromètre.

La source de lumière peut comprendre, par exemple, un laser supercontinuum qui émet une lumière avec un spectre large, par exemple dans le spectre visible et/ou infrarouge.

Le détecteur optique peut comprendre, par exemple, un élément dispersif tel qu'un réseau ou un prisme permettant de disperser spectralement la lumière, et un détecteur linéaire ou matriciel avec une pluralité de pixels ou détecteurs individuels permettant de mesurer l'intensité lumineuse séparément pour différentes longueurs d'onde.

Suivant d'autres modes de réalisation, le dispositif de l'invention peut comprendre une source de lumière du type d'un laser accordable ou à balayage, et un détecteur optique d'intensité.

Dans ce cas, le laser accordable ou à balayage émet une lumière monochromatique dont la longueur d'onde varie au cours du temps, pour réaliser une source polychromatique. L'intensité reçue sur le détecteur à chaque instant peut être reliée à la longueur d'onde d'émission pour reconstruire une mesure de densité spectrale de puissance.

Suivant des modes de mise en œuvre, le dispositif de l'invention peut comprendre une ligne à retard agencés de sorte à faire varier un trajet optique du faisceau optique de mesure, ou du faisceau optique de référence, ou de ces deux faisceaux (par exemple en sens opposé).

Suivant des modes de réalisation, le dispositif de l'invention peut comprendre une ligne à retard permettant d'introduire des délais optiques par pas discrets.

Dans ce cas les délais ou variations de trajets optiques introduits par la ligne à retard correspondent à un ensemble de valeurs discrètes.

La ligne à retard peut notamment comprend un commutateur optique.

Ce commutateur peut par exemple permettre de sélectionner des trajets optiques de longueur déterminée.

Suivant des modes de réalisation, le dispositif de l'invention peut comprendre une ligne à retard permettant d'introduire un délai optique continument variable.

Suivant des modes de réalisation, le dispositif de l'invention peut comprendre un interféromètre de Michelson.

Suivant d'autres modes de réalisation, le dispositif de l'invention peut comprendre un interféromètre à trajet commun avec un bras de mesure pour diriger le faisceau de mesure vers l'objet, et un élément semi-réfléchissant inséré dans ledit bras de mesure pour générer le faisceau optique de référence.

L'élément semi-réfléchissant peut comprendre tout élément apte à générer une réflexion partielle du faisceau incident. Il peut comprendre, de manière non limitative :

- un élément générant une discontinuité d'indice de réfraction entre deux portions de fibres optiques ;
- une interface fibre-air en extrémité de fibre optique ;
- un élément tel qu'une surface de verre ou une lame semi-réfléchissante inséré dans un trajet de faisceau en propagation libre ou dans l'air.

Le dispositif peut alors comprendre une ligne à retard différentielle avec une réflexion optique à une position fixe, et la ligne à retard.

Cette ligne à retard différentielle peut être agencée pour introduire un retard optique variable entre le faisceau optique de mesure et le faisceau optique de référence issus du bras de mesure.

Le dispositif peut également comprendre une ligne à retard différentielle avec une réflexion optique à une position variable, et la ligne à retard.

Suivant un autre aspect, il est proposé un procédé pour déterminer des informations sur la structure et/ou la localisation d'interfaces d'un objet mettant en œuvre un interféromètre à faible cohérence tel que défini par la revendication 9. Il comprend des étapes :

- d'émission d'une lumière polychromatique avec une source de lumière polychromatique ;
- de génération d'un faisceau optique de mesure réfléchi par ledit objet à mesurer, et d'un faisceau optique de référence ;

- d'introduction d'un délai optique variable entre le faisceau optique de mesure et le faisceau optique de référence avec une ligne à retard ;
- de combinaison, au moyen d'une détection optique, du faisceau optique de mesure et du faisceau optique de référence, et de production d'un signal spectral représentatif d'une densité spectrale de puissance optique du signal d'interférences résultant ;

Lequel procédé comprenant en outre des étapes :

- d'acquisition d'une pluralité de signaux spectraux pour une pluralité de délais optiques ;
- de détermination pour chaque signal spectral d'informations de retard optique entre faisceaux interférents dans une gamme de mesure dite spectrale ;
- d'analyse de l'évolution desdits retards optiques en fonction du délai optique et d'affectation du ou des retards optiques déterminés à partir des différents signaux spectraux à une ou des courbes dites d'interface, correspondant à des droites de pente unitaire positive ou négative, ou de pente nulle, en fonction du délai optique respectif de l'acquisition desdits signaux spectraux ; et
- de déduction d'informations sur la structure et/ou la localisation d'interfaces de l'objet en utilisant la ou les courbes d'interface.

Suivant des modes de mise en œuvre, le procédé de l'invention peut comprendre l'acquisition d'une pluralité de signaux spectraux pour une pluralité de délais optiques dans une gamme de délais optiques permettant de générer des égalités de parcours optiques entre le faisceau optique de référence et le faisceau optique de mesure lorsque l'objet se trouve dans une gamme de mesure dite temporelle.

Le procédé de l'invention peut ainsi par exemple comprendre l'acquisition d'une pluralité de signaux spectraux pour une pluralité de conditions de différences de trajets optiques entre le faisceau optique de référence et le faisceau optique de mesure tel que réfléchi par une interface particulière de l'objet, qui correspondent respectivement à une différence de trajets positive, puis nulle ou quasiment nulle, puis négative (ou inversement).

Suivant des modes de mise en œuvre, le procédé de l'invention peut comprendre l'acquisition d'une pluralité de signaux spectraux pour une pluralité de délais optiques espacés d'un incrément correspondant au plus à la moitié de la gamme de mesure spectrale.

Cette condition permet de détecter un retard optique entre mêmes faisceaux interférents au moins deux fois, pour deux valeurs consécutives de délais optiques.

La détermination d'informations de retard optique entre faisceaux interférents peut comprendre un calcul d'un signal d'interférences temporel, et la détermination de positions de pics d'interférences.

Le signal d'interférences temporel peut être par exemple déterminé en utilisant une transformée de Fourier, ou une transformée de Fourier inverse.

Des courbes d'interface correspondant à des droites de pente unitaire positive ou négative représentent en particulier l'évolution d'un retard optique lié à des interférences entre le faisceau optique de référence et le faisceau optique de mesure tel que réfléchi par une interface particulière de l'objet, puisque l'évolution du retard optique mesuré à partir des signaux spectraux correspond, à la valeur absolue près, à la variation de délai optique entre l'acquisition de ces signaux spectraux.

Selon l'invention, la structure et/ou la localisation d'interfaces de l'objet est déterminée en utilisant la ou les courbes d'interfaces.

En particulier, la structure et/ou la localisation d'interfaces de l'objet peut être déterminée en déterminant le ou les délais optiques respectifs pour lequel le retard optique le long d'une courbe d'interface est nul.

En effet, un délai optique qui respecte cette condition correspond à une condition pour laquelle il y a une égalité de trajets optiques entre le faisceau optique de référence et le faisceau optique de mesure tel que réfléchi par une interface particulière de l'objet. La connaissance du délai optique permet de localiser cette interface.

Les interfaces de l'objet sont ainsi localisées en termes de positions ou distances optiques. On peut ensuite déterminer les positions ou distances réelles (géométriques) en prenant en compte les indices de réfraction des milieux traversés.

Suivant un autre aspect, il est proposé un appareil comprenant un dispositif selon l'invention.

Il est notamment proposé un appareil selon l'invention pour mesurer des distances et/ou des épaisseurs de couches sur un objet comprenant des composants et/ou des éléments d'électronique intégrée et/ou d'optique intégrée.

L'objet peut être ou comprendre par exemple un wafer, et/ou des éléments de wafers, et/ou tout élément issu d'un procédé de fabrication de type microélectronique ou micro-optique.

Bien entendu, l'invention peut également être mise en œuvre pour effectuer des mesures sur tous types d'objets de nature industrielle ou biologique.

Il est ainsi également proposé un appareil selon l'invention pour mesurer des distances et/ou des épaisseurs de couches ou des structures de couches sur un objet biologique.

Cet objet peut être ou comprendre, par exemple, un tissu biologique, une culture cellulaire, de la peau, un vaisseau sanguin, ....

## Description des figures et modes de réalisation

D'autres avantages et particularités de l'invention apparaîtront à la lecture de la description détaillée de mises en œuvre et de modes de réalisation nullement limitatifs, et des dessins annexés suivants :

- la FIG. 1 illustre un premier mode de réalisation de dispositif selon l'invention,
- la FIG. 2 illustre un deuxième mode de réalisation de dispositif selon l'invention,
- la FIG. 3 illustre un mode de réalisation de ligne à retard utilisable dans un dispositif selon l'invention,
- la FIG. 4 illustre le principe de mesure de l'invention,
- la FIG. 5 illustre un exemple de mesures obtenues avec l'invention,
- la FIG. 6 illustre un organigramme du procédé de mesure selon l'invention.

Il est bien entendu que les modes de réalisation qui seront décrits dans la suite ne sont nullement limitatifs. L'invention est définie par les revendications.

Toutes les variantes et tous les modes de réalisation décrits sont combinables entre eux si rien ne s'oppose à cette combinaison sur le plan technique.

Sur les figures, les éléments communs à plusieurs figures conservent la même référence.

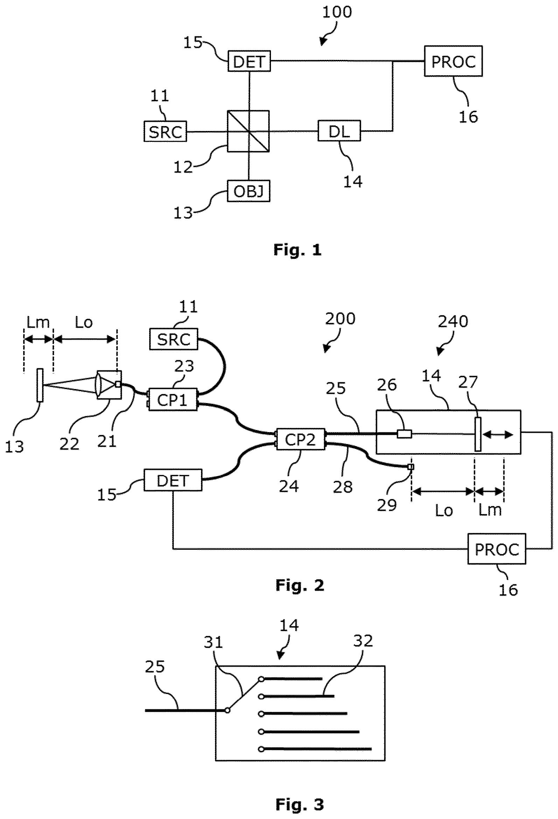

On va tout d'abord décrire, en référence à la FIG. 1, un premier mode de réalisation de dispositif selon l'invention.

Dans ce mode de réalisation, le dispositif 100 est réalisé sous la forme d'un interféromètre de Michelson. Il comprend une source de lumière 11, un élément optique séparateur 12, une ligne à retard temporelle 14, un détecteur 15 et une unité de traitement 16.

La lumière issue de la source de lumière 11 est divisée par l'élément séparateur 12 en un faisceau de mesure dirigé vers l'objet 13 et un faisceau de référence dirigé vers la ligne à retard 14. Ces deux faisceaux, après réflexion respectivement sur l'objet 13 et dans la ligne à retard 14, sont dirigés vers le détecteur 15 pour en mesurer les interférences.

Le dispositif 100 tel qu'illustré est réalisé avec des composants d'optique discrets, avec par exemple un élément optique séparateur 12 sous la forme d'un cube séparateur ou d'une lame semi-réfléchissante, et des faisceaux en propagation libre. Il peut bien entendu être réalisé avec toutes autres sortes de technologies ou de combinaisons de technologies, en utilisant par exemple des fibres optiques et/ou des guides planaires sur un substrat, et un élément optique séparateur 12 sous la forme par exemple d'un coupleur à fibres optiques ou de jonctions « Y » en guide planaires.

Le dispositif 100 met en œuvre une détection de type spectrale, qui permet d'obtenir en sortie du détecteur 15 un signal spectral représentatif d'une densité spectrale de puissance du signal optique incident sur ce détecteur 15.

Dans une configuration, le dispositif 100 comprend une source de lumière 11 à spectre large, qui peut être soit une source émettant de la lumière de manière continue (ASE, diode superluminescente), soit une source émettant de la lumière de manière impulsionnelle (laser supercontinuum). Dans cette configuration, le dispositif 100 comprend un détecteur 15 de type spectral (tel qu'un spectromètre), avec par exemple un élément dispersif qui permet de disperser spectralement la lumière sur un détecteur linéaire ou matriciel de sorte à produire une mesure représentative d'une densité spectrale de puissance optique.

Dans une autre configuration, le dispositif 100 comprend une source de lumière 11 accordable en longueur d'onde, par exemple de type laser accordable ou à balayage, donc la longueur d'onde d'émission peut être changée au cours du temps dans une gamme spectrale. Dans cette configuration, le dispositif 100 comprend un détecteur 15 d'intensité (tel qu'une photodiode, ...) qui mesure la puissance optique totale incidente. En faisant varier la longueur d'onde de la source de lumière 11, on obtient en sortie du détecteur 15 un signal représentatif d'une densité spectrale de puissance optique comme précédemment.

Le dispositif 100 comprend également une ligne à retard 14 qui permet de faire varier, de manière connue, la longueur du trajet optique du faisceau de référence relativement à celle du faisceau de mesure, et ainsi faire varier le délai optique ou la différence de trajets optiques entre les faisceaux de mesure et de référence. La présence de ce faisceau de référence permet de réaliser une référence optique à partir de laquelle les distances des différentes interfaces de l'objet 13 dont mesurées.

Dans une configuration, le dispositif 100 comprend une ligne à retard 14 qui permet de faire varier de manière continue au cours du temps le délai optique ou le trajet optique du faisceau de référence. Cette configuration peut être mise en œuvre avec une source de lumière 11 à large spectre, à émission continue ou impulsionnelle.

Dans une autre configuration, le dispositif 100 comprend une ligne à retard 14 qui permet de faire varier le délai optique ou le trajet optique du faisceau de référence par pas ou incréments discrets. Cette configuration peut être mise en œuvre avec tous types de sources de lumières 11.

Dans d'autres configurations non représentées, le dispositif 100 peut comprendre une ligne à retard qui permet de faire varier au cours du temps, de manière continue ou par pas ou incréments discrets, le délai optique ou le trajet optique du faisceau de mesure. Dans ce cas, le faisceau de référence peut être de longueur fixe, ou comprendre également une ligne à retard. La ligne à retard peut être réalisée par tous moyens, y compris un déplacement d'un collimateur de mesure, ou de l'ensemble de l'interféromètre avec l'élément optique séparateur 12, relativement à l'objet.

Comme expliqué précédemment, l'invention permet d'effectuer des mesures de distances ou d'épaisseur sur des grandes étendues de mesure, en bénéficiant des avantages d'une détection spectrale, notamment en termes de sensibilité et de disponibilité de sources. Pour cela on effectue une pluralité de mesures de signaux spectraux, pour une pluralité de délais optiques ou de positions de la ligne à retard. Ces mesures spectrales sont ensuite combinées par l'unité de traitement 16, en prenant en compte la position de la ligne à retard 14 où elles ont été réalisées (et donc le délai optique introduit), pour produire une mesure des épaisseurs et des positions respectives des couches de l'objet 13. Ce procédé d'acquisition et de traitement est décrit en détail plus loin.

On va maintenant décrire, en référence à la FIG. 2, un deuxième mode de réalisation de dispositif selon l'invention.

Dans ce mode de réalisation, le dispositif 200 est réalisé sous la forme d'un interféromètre à trajet commun (« common-path interferometer » en Anglais) ou de fizeau qui permet une mise en œuvre efficace de l'invention.

Le dispositif 200 est présenté dans un mode de réalisation à base de fibres optiques monomodes. Bien entendu il peut être réalisé avec tous types de composants, par exemple en propagation libre, ou avec des composants d'optique intégrée ou planaire, ou une combinaison de certaines de ces différentes techniques.

Le dispositif 200 peut être mis en œuvre dans les mêmes configurations, en termes de combinaisons de sources de lumière 11, de détecteurs 15 et de lignes à retard 14 que le dispositif 100 présenté en relation avec la FIG. 1.

Le dispositif 200 comprend un coupleur de codage 23 (qui peut aussi être un circulateur) qui dirige la lumière issue de la source de lumière 11 vers un bras de mesure. Ce bras de mesure comprend une fibre optique de mesure 21 et un collimateur de mesure 22 permettant de collimater ou focaliser un faisceau de mesure sur l'objet 13.

Le faisceau incident dans la fibre optique de mesure 21 est partiellement réfléchi au niveau du collimateur de mesure 22, par exemple en exploitant la réflexion de Fresnel au bout de la fibre de mesure 21, pour générer un faisceau de référence. La réflexion en bout de fibre peut également être ajustée en y appliquant un dépôt semi-réfléchissant, diélectrique ou métallique.

Le faisceau de mesure tel que réfléchi par l'objet 13 et le faisceau de référence sont dirigée par le coupleur de codage 23 vers un coupleur de décodage 24. Ils sont ensuite dirigés vers une ligne à retard différentielle 240 avec un bras de référence de longueur fixe 28 terminé par un élément réfléchissant fixe 29 (constitué par exemple par un miroir ou un dépôt réfléchissant placé en bout de fibre optique), et un bras de longueur variable 25 avec une ligne à retard 14. Chacun des faisceaux de mesure et de référence est dirigé pour partie dans le bras de référence 28 et pour partie dans le bras de longueur variable 25 de la ligne à retard différentielle 240.

Les faisceaux réfléchis respectivement dans le bras de référence 28 et dans le bras de longueur variable 25 sont ensuite dirigés par le coupleur de décodage 24 vers le détecteur 15.

Dans le mode de réalisation présenté, la ligne à retard 14 comprend un élément réfléchissant mobile 27 sous la forme d'un miroir mobile 27, et un collimateur 26 pour collimater ou focaliser la lumière issue de la fibre optique sur le miroir mobile 27. Le miroir mobile 27 est déplacé en translation, entre une position proximale vers le collimateur 26 et une position distale à l'opposé, par un système de motorisation avec des moyens (tels qu'une règle optique) pour mesurer précisément sa position.

Suivant les configurations, le miroir mobile 27 peut être déplacé de manière continue ou par pas discrets.

Suivant une variante, l'élément réfléchissant fixe 29 peut être réalisé par une réflexion partielle dans le bras de longueur variable 25, telle que par exemple une réflexion en bout de fibre au niveau du collimateur 26 de la ligne à retard 14. Dans ce cas, les deux bras de la ligne à retard différentielle 240 sont confondus.

La ligne à retard différentielle 240 permet de moduler ou compenser les différences de trajets optiques introduites entre les faisceaux de mesure et de référence dans le bras de mesure.

Le mode de réalisation du dispositif 200 présente des avantages par rapport à l'interféromètre de Michelson simple du dispositif 100 illustré à la FIG. 1. En particulier, les faisceaux de mesure et de référence parcourent le même trajet dans la fibre optique de mesure 21, de sorte que la longueur de cette fibre et les perturbations qu'elle subit n'affectent pas les mesures.

Le dispositif 200 permet également d'effectuer des mesures de distances ou d'épaisseur sur des grandes étendues de mesure, en bénéficiant des avantages d'une détection spectrale, notamment en termes de sensibilité et de disponibilité de sources. Pour cela on effectue une pluralité de mesures de signaux spectraux, pour une pluralité de positions ou de délais optiques de la ligne à retard 14. Ces signaux spectraux sont ensuite combinés par l'unité de traitement 16, en prenant en compte la position de la ligne à retard 14 où elles ont été réalisées (ou le délai optique correspondant), pour produire une mesure des épaisseurs et des positions respectives des couches de l'objet 13. Ce procédé d'acquisition et de traitement est décrit en détail plus loin.

Suivant des variantes, les dispositifs 100 et 200 décrits précédemment peuvent comprendre tous types de lignes à retard 14 permettant de générer des délais optiques ou des variations de trajets optiques, tels que par exemple :

- une ligne à retard basée sur une translation mécanique (ou une oscillation) comme illustrée à la FIG. 2, qui permet de réaliser des variations de trajets optiques continues ou par pas discrets ;
- une ligne à retard basée sur une rotation d'un élément réfléchissant ou d'un élément transparent, tel qu'un polygone à faces parallèles inséré dans le trajet d'un faisceau optique, qui permet de réaliser des variations de trajets optiques continues ou par pas discrets ;

- une ligne à retard basée sur une translation ou une rotation d'un élément dont la forme permet de faire varier un trajet optique en fonction du déplacement, de manière continue ou par pas discrets ;

- une ligne à retard avec une pièce en escalier comprenant une succession de faces planes, parallèles entre elles, décalées dans la direction du faisceau, et qui est déplacée dans une direction perpendiculaire au faisceau de sorte à positionner séquentiellement les faces devant le faisceau et réaliser ainsi des variations de trajets optiques par pas discrets.

La FIG. 3 illustre un mode de réalisation de ligne à retard 14 permettant de générer des variations de trajets optiques ou de délais optiques par pas discrets.

La ligne à retard 14 comprend un commutateur optique 31 et une pluralité de canaux optiques 32 de longueur différente qui réfléchissent la lumière à leur extrémité. Le commutateur optique 31 permet de diriger la lumière incidente dans un canal 32 particulier, de sorte à lui faire parcourir un trajet de longueur déterminée. En commutant le canal sélectionné, on peut ainsi changer le trajet optique du faisceau, ou le délai optique correspondant.

De préférence le commutateur optique 31 est un composant fibré.

Les canaux optiques 32 peuvent être réalisés par exemple avec des fibres optiques avec une terminaison réfléchissante.

Les canaux optiques 32 peuvent également être réalisés avec des guides d'ondes planaires sur un substrat optique. Ce mode de réalisation a l'avantage de permettre un ajustement précis de la longueur des canaux optiques par construction. Leur trajet sur la surface du substrat peut être ajusté de sorte qu'ils terminent tous sur une tranche du substrat qui est métallisée pour générer une réflexion en bout de guide.

Les canaux optiques peuvent également être réalisés en propagation libre. Dans ce cas, chaque canal optique peut comprendre une fibre optique, une lentille de collimation et un réflecteur placé à la distance voulue.

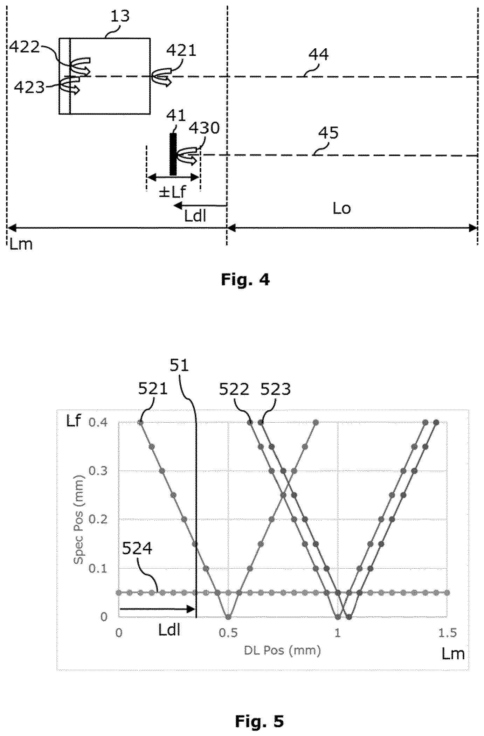

La FIG. 4 illustre le principe de mesure de l'invention, tel qu'il peut être mis en œuvre par exemple avec les dispositifs 100 et 200.

Plus précisément, la FIG. 4 illustre de manière schématique les longueurs des trajets optiques respectifs d'un faisceau de mesure 44 et d'un faisceau de référence 45, tels qu'ils se propagent entre la source optique 11 et le détecteur 15 dans les dispositifs 100 et 200.

Dans l'exemple illustré, le faisceau de mesure 44 incident sur un objet 13 subit des réflexions 421, 422, 423 sur les interfaces de l'objet. Le faisceau de référence 45 est réfléchi sur une surface de référence équivalente 41.

Dans les modes de réalisation des dispositifs 100 et 200, la position de la surface de référence équivalente 41 représente un trajet optique dans la ligne à retard 14. Dans cette représentation, la ligne à retard 14 permet d'introduire dans le faisceau de référence 45 un délai optique correspondant à un trajet optique $Ldl$ qui peut varier entre une valeur par exemple nulle (sans perte de généralité) et une valeur maximale $Lm$ correspondant à la course maximale de la ligne à retard. Dans le cas d'une ligne à retard avec un miroir mobile 27 en translation telle qu'illustrée à la FIG. 2, le trajet optique $Ldl$ peut être obtenu en déplaçant le miroir mobile d'une distance correspondante depuis sa position proximale.

Pour simplifier la description, les trajets ou délais optiques ne sont comptés que sur un aller (ou retour) des faisceaux, de sorte à correspondre aux dimensions ou déplacements optiques des éléments qui génèrent les réflexions. En pratique les faisceaux de mesure et de référence parcourent des trajets aller-retour, donc doubles, mais le fait de ne considérer que des demi trajets ne modifie pas les conditions d'équilibre.

Plus généralement, la longueur du trajet optique de référence 45 est ajustée de sorte à pouvoir varier, en fonction du déplacement de la ligne à retard, entre une distance minimale dite d'offset $Lo$ et la distance maximale $Lo + Lm,$ correspondant à la distance d'offset additionnée de la course de la ligne à retard. Cette gamme de distances optiques entre la distance d'offset $Lo$ et la distance maximale $Lo + Lm$ correspond, pour le faisceau de mesure 44, à la gamme de mesure de distances temporelle du dispositif, car il n'est possible d'obtenir des égalités de chemins optiques entre le faisceau de référence 45 et le faisceau de mesure 44 que pour des réflexions sur l'objet 13 ayant lieux dans cette gamme de mesure de distances temporelle.

De manière générale, la distance d'offset $Lo$ peut être définie par rapport à un élément qui sépare les trajets des faisceaux de mesure et de référence. Elle dépend de l'équilibrage des différents bras du ou des interféromètres.

Par exemple, dans le dispositif 100, la distance d'offset $Lo$ peut être définie par rapport à l'élément séparateur 12. Elle dépend de la longueur optique du bras de référence, ou en d'autres termes de la distance optique parcourue par le faisceau de référence entre l'élément séparateur 12 et la ligne à retard temporelle 14.

Dans le dispositif 200, la distance d'offset $Lo$ peut être défini par rapport à l'élément qui génère le faisceau de référence dans le bras de mesure, au niveau du collimateur de mesure 21. Elle dépend par contre de la différence de trajets optiques entre le bras de référence de longueur fixe 28 et le bras de longueur variable 25 de la ligne à retard différentielle 240. En effet, comme expliqué précédemment, chacun des faisceaux de mesure et de référence est séparé en deux composantes par le coupleur de décodage 24.

Dans le mode de réalisation illustré, la ligne à retard différentielle 240 est équilibrée ou agencée de sorte que le trajet optique le long de la composante du faisceau de référence réfléchie par le miroir mobile 27 jusqu'au détecteur 15 puisse correspondre à un trajet optique le long d'une composante d'un faisceau de mesure réfléchi par l'élément réfléchissant fixe 29 qui serait généré par une réflexion sur l'objet dans la gamme de mesure de distances temporelle du dispositif *[Lo ; Lo+Lm]*.

Alternativement, la ligne à retard différentielle 240 peut être équilibrée ou agencée de sorte que le trajet optique le long de la composante du faisceau de référence réfléchie par l'élément réfléchissant fixe 29 jusqu'au détecteur 15 puisse correspondre à un trajet optique le long d'une composante d'un faisceau de mesure réfléchi par le miroir mobile 27 qui serait généré par une réflexion sur l'objet dans la gamme de mesure de distances temporelle du dispositif *[Lo ; Lo+Lm]*.

Dans les dispositifs de l'art antérieur qui mettent en œuvre une détection temporelle, on obtiendrait un pic d'interférences (ou une bouffée d'interférences) sur le détecteur pour chaque position de la ligne à retard *Ldl* pour laquelle le trajet optique du faisceau de référence 45 jusqu'à la surface de référence équivalente 41 correspond à un trajet optique du faisceau de mesure 44 jusqu'à une interface de l'objet 13. On obtiendrait donc un pic d'interférence pour chaque interface de l'objet 13 situés dans la gamme de mesure de distances temporelle du dispositif *[Lo ; Lo+Lm]*. La largeur de ces pics d'interférences serait fonction de la longueur de cohérence de la source.

Comme expliquée précédemment, l'invention met en œuvre une détection spectrale ou fréquentielle. On détecte donc en sortie du détecteur 15 un signal spectral représentatif d'une densité spectrale de puissance optique. Ce signal spectral dépend du spectre de la source, et des interférences entre couples de faisceaux qui se superposent sur le détecteur. En appliquant le théorème de Wiener-Khinchin, on peut calculer une transformée de Fourier inverse de ce signal spectral et ainsi obtenir sa fonction d'autocorrélation dans le domaine temporel ou en distance. On obtient ainsi un signal d'interférences temporel en distances optiques ou en retards optiques avec un pic d'interférence pour chaque différence de trajet optique ou chaque retard optique entre des couples de faisceaux incidents sur le détecteur, dans une gamme de mesure spectrale de distances optiques *Lf.*

Comme cela a été expliqué précédemment, cette gamme de mesure spectrale correspond, en distances optiques, au maximum à :

$$Lfmax = N/2 \, l_c,$$

Avec $l_c$ la longueur de cohérence de la source et *N* le nombre de points d'échantillonnage du spectre. Elle est donc directement limitée par le nombre de points de mesure du spectromètre ou, avec une source accordable, par le nombre de longueur d'ondes distinctes qui peuvent être générées.

La détection spectrale n'est pas sensible au signe des différences de trajets optiques mesurées. Elle en fournit donc que la valeur absolue (ou une valeur non signée). Ainsi, pour une position *Ldl* de la surface de référence équivalente 41 (ou un délai optique introduit par la ligne à retard), la détection spectrale fournir une mesure, en valeur absolue, des différences de trajets optiques ou des retards optiques entre le faisceau de référence 45 et les réflexions du faisceau de mesure 44 sur des interfaces de l'objet se trouvant à des distances optiques *Lo + Ldl ± Lf.*

La détection spectrale est également sensible à des différences de trajets optiques entre réflexions du faisceau de mesure. Elle produit donc un signal ambigu, compliqué à analyser dans le cas général.

Pour résoudre les ambiguïtés de signe et distinguer les interférences entre réflexions du faisceau de mesure 44 de celles entre le faisceau de référence 45 et le faisceau de mesure 44, une solution de l'art antérieur consiste à positionner de manière statique la surface de référence équivalente 41 de sorte que les différences de trajets optiques entre le faisceau de référence 45 et les réflexions du faisceau de mesure 44 sur des interfaces de l'objet 13 (ou les retards optiques correspondants) aient toutes le même signe, et une valeur supérieure aux épaisseurs entre couches de l'objet 13. Cela revient à régler la ligne à retard de sorte que les trajets optiques le long du faisceau de mesure 44 jusqu'aux réflexions 421, 422, 423 soient tous supérieurs (ou inférieurs) au trajet optique le long du faisceau de référence 45 jusqu'à la réflexion 430 sur la surface de référence équivalente 41. Mais cela limite fortement la gamme de mesure de distances exploitable, qui correspond au maximum à la gamme de mesure spectrale de distances optiques *Lf.*

La solution de l'invention consiste à acquérir une pluralité de mesures spectrales ou de signaux spectraux à une pluralité de positions de la surface de référence équivalente 41 correspondant à des distances *Ldl* (ou des délais optiques) comprises dans la gamme de mesure de distances temporelle du dispositif *[Lo ; Lo+Lm]*. On peut alors déterminer de manière non ambiguë la structure de l'objet à partir des courbes dites « d'interface » parcourues par les pics d'interférences détectés dans les signaux spectraux acquis successivement.

La FIG. 5 illustre des exemples de mesures obtenues dans ces conditions, dans la configuration de mesure illustrée à la FIG. 4.

L'axe horizontal « DL Pos » correspond à des différences de trajets optiques (ou des délais optiques) générées par la ligne à retard, ou dans l'exemple illustré à la FIG. 4, à la position *Ldl* de la surface de référence équivalente 41. L'axe vertical « Spec Pos » correspond à des différences de trajets optiques (ou des retards optiques) obtenues à partir des signaux spectraux tels que mesurés par le détecteur spectral 15, dans la gamme de mesure de distances spectrales optiques *Lf*. On construit ainsi un diagramme mesures spectrales vs. positions de référence ou délais optiques.

Comme expliqué précédemment, la position *Ldl* de la surface de référence équivalente 41 est déplacée dans la gamme de mesure de distances temporelle du dispositif *[Lo ; Lo+Lm]* dans laquelle se trouvent les interfaces de l'objet 13 selon une pluralité de positions de mesure 51 correspondant à des délais optiques de la ligne à retard. Pour chacune de ces positions de mesure 51, on effectue une mesure de signal spectral dont on déduit des positions de pics d'interférences correspondant à des mesures de retards optiques.

Les différents retards optiques mesurés s'inscrivent dans le diagramme mesures de signaux spectraux vs. positions ou délais optiques sous la forme de courbes d'interface dont la forme dépend de l'origine du retard optique.

Ainsi, par exemple, une couche mince dont l'épaisseur est inférieure à l'étendue de mesures spectrales *Lf* donne lieu à un pic d'interférences 524, issu de l'interférence entre les deux réflexions 422, 423 du faisceau de mesure 44, dont la position dans le diagramme est indépendante de la position de mesure 51 de la surface de référence équivalente 41.

Par ailleurs, lorsqu'on a une différence de trajets optiques entre le faisceau de mesure 44 réfléchi sur une interface de l'objet 13 et le faisceau de référence 45 inférieure à l'étendue de mesures spectrales *Lf,* on obtient un pic d'interférence dont la position dans le diagramme dépend de la valeur absolue de la différence de trajets optiques (ou du retard optique correspondant). Par exemple, les interférences entre la réflexion 430 du faisceau de référence 45 sur la surface de référence et, respectivement, les réflexions 421, 422, 423 du faisceau de mesure 44 sur les interfaces de l'objet 13 donnent lieu à des pics d'interférences 521, 522, 523. Ces pics d'interférences s'inscrivent donc sur des courbes d'interfaces en forme de « V » qui correspondent à des droites de pente ±1 en distances optiques (ou en retards optiques). Ces droites correspondant à une interface de l'objet se coupent sur l'origine des signaux spectraux (position nulle) ou sur l'axe des délais optiques, ce qui correspond à la condition selon laquelle la surface de référence équivalente 41 est à la même distance optique que l'interface de l'objet. En d'autres termes, la position de l'intersection entre une courbe ou une droite d'interface et l'axe des délais optiques correspond au délai optique pour lequel on a une égalité de chemin optique entre le faisceau de référence et de faisceau de mesure réfléchi par l'interface de l'objet correspondant à cette courbe d'interface.

On peut noter qu'il n'est pas possible dans ces conditions de déterminer la structure d'un objet 13 dans le cas général, même dans un voisinage correspondant à l'étendue de mesure spectrale *Lf,* avec une seule mesure spectrale réalisée pour un seul délai optique ou avec une surface de référence équivalente positionnée à une seule position de mesure 51.

Suivant l'invention, on effectue des mesures spectrales pour une pluralité de positions de mesures 51 de la surface de référence équivalente 41 (ou de positions ou délais optiques de la ligne à retard) de sorte que chaque pic d'interférences ou chaque retard optique mesuré puisse être affecté à une courbe d'interface sous la forme d'une droite. Pour cela, il faut que chaque pic d'interférences soit détecté au moins deux fois le long de cette droite. Cette condition est respectée dès lors que l'incrément de position *ΔLdl* (ou la différence de délai optique correspondante) entre des positions de mesure 51 successives de la surface de référence équivalente 41 est inférieur ou égal à la moitié de la gamme de mesure spectrale *Lf* exploitée.

L'affectation des pics d'interférences aux différentes courbes ou droites d'interface est simplifiée par le fait que les pics d'interférences de l'objet ne peuvent se trouver que sur des droites de pente +1, -1 ou nulle.

Les pics d'interférences situés sur des droites de pentes positives ou négatives correspondent à des positions d'interfaces de l'objet mesurées relativement aux positions de la surface de référence équivalente 41. Le signe de la pente de la droite indique le signe de cette différence de position. Ainsi, en connaissant la position de la surface de référence équivalente 41 (ou le délai optique correspondant) pour chaque point de mesure 51, on peut localiser sans ambiguïté la position correspondante de l'interface de l'objet dans la gamme de mesure de distances temporelle du dispositif *[Lo ; Lo+Lm]*.

Les pics d'interférences situés sur des droites d'interface de pentes nulles correspondent à des différences de positions d'interfaces de l'objet, mais ne fournissent pas d'information sur la localisation de ces interfaces. Elles doivent donc être distinguées pour éviter des fausses détections. Cette discrimination peut être effectuée à partir de la valeur de leur pente.

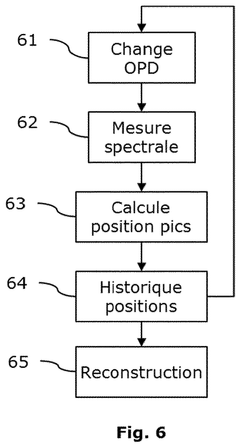

La FIG. 6 illustre un exemple d'organigramme du procédé selon l'invention. Il comprend :

- Une étape 61 de positionnement de la ligne à retard pour positionner la surface de référence équivalente 41 à une position de mesure 51 connue, correspondant à une valeur de délai optique ;
- Une étape 62 d'acquisition d'un signal spectral ;
- Une étape 63 de calcul de la position des pics d'interférence ou des retards optiques correspondants dans le signal spectral ;
- Une étape 64 de stockage des positions de pics d'interférences ou des retards optiques correspondants dans un historique de mesures ;
- Une étape 65 de reconstruction de la structure de l'objet.

Comme expliqué précédemment, l'étape de reconstruction comprend une affectation des positions des pics d'interférences (ou des retards optiques correspondants) à des courbes d'interface, et/ou une construction de ces courbes d'interfaces.

Pour cela, par exemple, pour chaque nouveau signal spectral mesuré, on essaie d'affecter les pics d'interférences détectés à des droites existantes (à partir de la distance de ces pics à ces droites), et/ou on vérifie si on peut créer des nouvelles droites avec ces pics d'interférence et des pics d'interférences détectés lors d'une mesure spectrale précédents et non affectés à une droite.

On obtient ainsi un ensemble de droites ou de courbes d'interface. Pour obtenir la position des interfaces de l'objet, on peut par exemple déterminer la position de l'intersection de chaque droite d'interface de pente non-nulle avec l'axe temporel des positions de mesure 51 (ou des délais optiques). Comme expliqué précédemment, cette intersection correspond à la position de l'interface recherchée (ou au retard optique correspondant). On peut également, ou en complément, déterminer la position sur l'axe temporel ou des délais optiques de l'intersection entre deux droites de pente opposée se croisant sur cet axe et correspondant à une même interface.

L'invention présente l'avantage de permettre une grande précision de mesure. En effet, la position de chaque interface est déterminée à partir de plusieurs mesures, ce qui permet une amélioration significative du rapport signal à bruit et de l'incertitude de mesure, aussi bien en ce qui concerne la détection du signal que la prise en compte des incertitudes de mesure de la détection spectrale et de la mesure de position de la ligne à retard.

En outre, en particulier lorsqu'on utilise des sources impulsionnelles, grâce à la mise en œuvre de la détection spectrale le dispositif n'est pas sensible au bruit d'intensité de la source ou à la variation d'intensité d'une impulsion à l'autre.

L'invention présente également l'avantage de permettre la réalisation de dispositifs avec simultanément :

- une grande étendue de mesure atteignable uniquement avec une détection temporelle de type TD-OCR, et
- une épaisseur minimale mesurable très fine, rendue possible par la possibilité de mettre en œuvre des sources impulsionnelles de plus large spectre et de puissance supérieure à ce qui peut être atteint avec des sources continues, ou des sources à balayage de fréquence.

A titre d'exemple non limitatif, l'invention peut être mise en œuvre pour réaliser un système de type OCR qui permette de mesurer des épaisseurs et des positions d'interfaces d'objets avec une épaisseur ou une distance optique maximale mesurable de 10 mm, une épaisseur ou une distance optique minimale mesurable inférieure à 5 μm, et une cadence de mesure de 100Hz.

Pour cela, on peut mettre en œuvre un dispositif 100 ou 200 selon l'invention avec :

- une source de lumière 11 de type laser supercontinuum qui produit une lumière avec un spectre de largeur supérieur à 200 nm autour de 1310nm, sous la forme d'impulsions nanosecondes avec une fréquence de répétition de l'ordre 10 KHz ;
- une ligne à retard 14 avec un miroir en déplacement linéaire oscillatoire sur une course de 10 mm à 50 Hz, soit un déplacement à une vitesse moyenne de 1 m/s ;
- un détecteur optique 15 avec un élément dispersif et un capteur linéaire.

Dans ces conditions, la source de lumière a une longueur de cohérence inférieure à 5 μm, et permet donc d'atteindre des épaisseurs ou distances optiques minimales mesurables inférieure à 5 μm. Le détecteur optique permet, comme expliqué précédemment, de mesurer des épaisseurs ou des distances optiques dans une gamme de mesure spectrale s'étendant de 5 μm à 500 μm. En supposant qu'on acquiert un signal spectral pour chaque impulsion de la source, soit à une fréquence de 10 KHz, on obtient un signal spectral tous les 100 μm de déplacement de la ligne à retard. Et donc chaque interface de l'objet peut être est détectée potentiellement jusqu'à cinq fois dans cinq signaux spectraux consécutifs, et ce dans toute la course de la ligne à retard, soit 10 mm.

Il est à noter qu'une telle étendue de mesure de 5 μm à 10 mm n'est pas réalisable par un système spectral (FD-OCT ou SS-OCT) de l'art antérieur à cause des besoins en échantillonnage du spectre et des limitations en longueur de cohérence de la source.

Par ailleurs, une épaisseur optique maximale mesurable de 10 mm est atteignable avec un système temporel de type TD-OCR de l'art antérieur. Mais pour échantillonner le signal d'interférence temporel avec une ligne à retard qui se déplace dans notre exemple à 1 m/s, il faut utiliser une fréquence d'échantillonnage de 15 MHz ou plus pour obtenir de l'ordre de 10 points par frange à 1310 nm de longueur d'onde. Une source continue est donc nécessaire. Or, les sources disponibles telles que les diodes super luminescentes (SDL) ont au mieux une largeur spectrale de l'ordre de 100 nm à 1310 nm, et ne permettent pas d'atteindre des épaisseurs optiques minimales mesurables inférieures à 10 μm, ce qui reste insuffisant.

Bien sûr, l'invention n'est pas limitée aux exemples qui viennent d'être décrits et de nombreux aménagements peuvent être apportés à ces exemples sans sortir du cadre de l'invention, qui est défini par les revendications suivantes.

**Revendications**

1. Dispositif interféromètre à faible cohérence (100, 200) pour déterminer des informations sur la structure et/ou la localisation d'interfaces d'un objet (13) comprenant :

- une source de lumière (11) polychromatique ;
- un système optique pour générer un faisceau optique de mesure réfléchi par ledit objet (13), et un faisceau optique de référence ;
- une ligne à retard (14) pour introduire un délai optique variable entre le faisceau optique de mesure et le faisceau optique de référence ;
- une détection optique (15) pour combiner le faisceau optique de mesure et le faisceau optique de référence, et produire un signal spectral représentatif d'une densité spectrale de puissance optique du signal d'interférences résultant;
**caractérisé en ce qu'**il comprend en outre un module de contrôle et de traitement (16) agencé pour :
- acquérir une pluralité de signaux spectraux pour une pluralité de délais optiques ;
- déterminer pour chaque signal spectral des informations de retard optique entre faisceaux interférents dans une gamme de mesure dite spectrale ;
- analyser l'évolution desdits retards optique en fonction du délai optique et affecter le ou les retards optiques déterminés à partir des différents signaux spectraux à une ou des courbes dites d'interface (521, 522, 523, 524), correspondant à des droites de pente unitaire positive ou négative ou de pente nulle, en fonction du délai optique respectif de l'acquisition desdits signaux spectraux ; et
- en déduire des informations sur la structure et/ou la localisation d'interfaces de l'objet (13) en utilisant la ou les courbes d'interfaces (521, 522, 523, 524).

2. Le dispositif de la revendication 1, qui comprend une source de lumière (11) émettant une lumière polychromatique sous forme d'impulsions et un détecteur optique (15) de type spectral.

3. Le dispositif de la revendication 1, qui comprend une source de lumière (11) avec un laser accordable ou à balayage, et un détecteur optique (15) d'intensité.

4. Le dispositif de l'une des revendications 1 à 3, qui comprend une ligne à retard (14) permettant d'introduire des délais optiques par pas discrets.

5. Le dispositif de la revendication 4, dans lequel la ligne à retard (14) comprend un commutateur optique (31).

6. Le dispositif de l'une quelconque des revendications 1 ou 2, qui comprend une ligne à retard (14) permettant d'introduire un délai optique continûment variable.

7. Le dispositif de l'une des revendications précédentes, qui comprend un interféromètre à trajet commun avec un bras de mesure (21, 22) pour diriger le faisceau de mesure vers l'objet, et un élément semi-réfléchissant inséré dans ledit bras de mesure pour générer le faisceau optique de référence.

8. Le dispositif de la revendication 7, qui comprend une ligne à retard différentielle (240) avec une réflexion optique (29) à une position fixe, et la ligne à retard (14).

9. Procédé pour déterminer des informations sur la structure et/ou la localisation d'interfaces d'un objet mettant en œuvre un interféromètre à faible cohérence (100, 200), comprenant des étapes :

- d'émission d'une lumière polychromatique avec une source de lumière (11) polychromatique ;
- de génération d'un faisceau optique de mesure réfléchi par ledit objet (13) à mesurer, et d'un faisceau optique de référence ;
- d'introduction d'un délai optique variable entre le faisceau optique de mesure et le faisceau optique de référence avec une ligne à retard (14) ;
- de combinaison, au moyen d'une détection optique (15), du faisceau optique de mesure et du faisceau optique de référence, et de production d'un signal spectral représentatif d'une densité spectrale de puissance optique du signal d'interférences résultant ;
- d'acquisition d'une pluralité de signaux spectraux pour une pluralité de délais optiques ;
- de détermination pour chaque signal spectral d'informations de retard optique entre faisceaux interférents dans une gamme de mesure dite spectrale ;
- d'analyse de l'évolution desdits retards optiques en fonction du délai optique;
**caractérisé en ce qu'**il comprend en outre des étapes:
d'affectation du ou des retards optiques déterminés à partir des différents signaux spectraux à une ou des courbes dites d'interface (521, 522, 523, 524), correspondant à des droites de pente unitaire positive ou négative ou de pente nulle, en fonction du délai optique respectif de l'acquisition desdits signaux spectraux ; et
- de déduction d'informations sur la structure et/ou la localisation d'interfaces de l'objet (13) en utilisant la ou les courbes d'interface (521, 522, 523, 524).

10. Le procédé de la revendication 9, qui comprend l'acquisition d'une pluralité de signaux spectraux pour une pluralité de délais optiques dans une gamme de délais optiques permettant de générer des égalités de parcours optiques entre le faisceau optique de référence et le faisceau optique de mesure lorsque l'objet (13) se trouve dans une gamme de mesure dite temporelle.

11. Le procédé de l'une quelconque des revendications 9 ou 10, qui comprend l'acquisition d'une pluralité de signaux spectraux pour une pluralité de délais optiques espacés d'un incrément correspondant au plus à la moitié de la gamme de mesure spectrale.

12. Le procédé de l'une des revendications 9 à 11, dans lequel la détermination d'informations de retard optique entre faisceaux interférents comprend un calcul d'un signal d'interférences temporel, et la détermination de positions de pics d'interférences.

13. Le procédé de l'une des revendications 9 à 12, dans lequel la structure et/ou la localisation d'interfaces de l'objet (13) est déterminée en déterminant le ou les délais optiques respectifs pour lequel le retard optique le long d'une courbe d'interface est nul.

14. Appareil comprenant un dispositif selon l'une quelconque des revendications 1 à 8.

**Patentansprüche**

1. Niederkohärenz-Interferometervorrichtung (100, 200) zum Bestimmen von Informationen über die Struktur und/oder die Lage von Grenzflächen eines Objekts (13), umfassend:

   - eine polychromatische Lichtquelle (11);
   - ein optisches System zum Erzeugen eines optischen Messstrahls, der durch das Objekt (13) reflektiert wird, und eines optischen Referenzstrahls;
   - eine Verzögerungsleitung (14) zum Einführen einer variablen optischen Verzögerung zwischen dem optischen Messstrahl und dem optischen Referenzstrahl;
   - eine optische Erfassung (15) zum Kombinieren des optischen Messstrahls und des optischen Referenzstrahls und zum Erzeugen eines Spektralsignals, das für eine optische Leistungsspektraldichte des resultierenden Interferenzsignals repräsentativ ist;
   **dadurch gekennzeichnet, dass** sie ferner ein Steuer- und Verarbeitungsmodul (16) umfasst, das eingerichtet ist zum:

      - Erfassen einer Vielzahl von Spektralsignalen für eine Vielzahl von optischen Verzögerungen;
      - Bestimmen, für jedes Spektralsignal, von optischen Verzögerungsinformationen zwischen interferierenden Strahlen in einem sogenannten spektralen Messbereich;
      - Analysieren der Entwicklung der optischen Verzögerungen in Abhängigkeit von der optischen Verzögerung und zum Zuordnen der anhand der verschiedenen Spektralsignale bestimmten optischen Verzögerung oder Verzögerungen zu einer oder mehreren sogenannten Grenzflächenkurven (521, 522, 523, 524), die Geraden mit einheitlicher positiver oder negativer Steigung oder Null-Steigung entsprechen, in Abhängigkeit von der jeweiligen optischen Verzögerung der Erfassung der Spektralsignale; und
      - Ableiten hieraus von Informationen über die Struktur und/oder die Lage von Grenzflächen des Objekts (13) unter Verwendung der Grenzflächenkurve oder -kurven (521, 522, 523, 524).

2. Vorrichtung nach Anspruch 1, die eine Lichtquelle (11), welche ein polychromatisches Licht in Form von Impulsen emittiert, sowie einen optischen Detektor (15) vom Spektraltyp umfasst.

3. Vorrichtung nach Anspruch 1, die eine Lichtquelle (11) mit einem abstimmbaren oder scannenden Laser sowie einen optischen Intensitätsdetektor (15) umfasst.

4. Vorrichtung nach einem der Ansprüche 1 bis 3, die eine Verzögerungsleitung (14) umfasst, welche ermöglicht, optische Verzögerungen in diskreten Schritten einzuführen.

5. Vorrichtung nach Anspruch 4, bei der die Verzögerungsleitung (14) einen optischen Schalter (31) umfasst.

6. Vorrichtung nach einem der Ansprüche 1 oder 2, die eine Verzögerungsleitung (14) umfasst, welche ermöglicht, eine kontinuierlich variable optische Verzögerung einzuführen.

7. Vorrichtung nach einem der vorhergehenden Ansprüche, die ein Interferometer mit gemeinsamem Weg mit einem Messarm (21, 22), um den Messstrahl in Richtung des Objektes zu lenken, sowie einem in den Messarm eingesetzten halbreflektierenden Element umfasst, um den optischen Referenzstrahl zu erzeugen.

8. Vorrichtung nach Anspruch 7, die eine Differenzverzögerungsleitung (240) mit einer optischen Reflexion (29) an einer festen Position und die Verzögerungsleitung (14) umfasst.

9. Verfahren zum Bestimmen von Informationen über die Struktur und/oder die Lage von Grenzflächen eines Objekts, das ein Niederkohärenz-Interferometer (100, 200) verwendet, umfassend die Schritte:

- des Emittierens eines polychromatischen Lichts mit einer polychromatischen Lichtquelle (11);
- des Erzeugens eines optischen Messstrahls, der durch das zu messende Objekt (13) reflektiert wird, und eines optischen Referenzstrahls;
- des Einführens einer variablen optischen Verzögerung zwischen dem optischen Messstrahl und dem optischen Referenzstrahl mit einer Verzögerungsleitung (14);
- des Kombinierens, mittels einer optischen Erfassung (15), des optischen Messstrahls und des optischen Referenzstrahls und des Erzeugens eines Spektralsignals, das für eine optische Leistungsspektraldichte des resultierenden Interferenzsignals repräsentativ ist;
- des Erfassens einer Vielzahl von Spektralsignalen für eine Vielzahl von optischen Verzögerungen;
- des Bestimmens, für jedes Spektralsignal, von optischen Verzögerungsinformationen zwischen interferierenden Strahlen in einem sogenannten spektralen Messbereich;
- des Analysierens der Entwicklung der optischen Verzögerungen in Abhängigkeit von der optischen Verzögerung;
**dadurch gekennzeichnet, dass** es ferner Schritte umfasst:

- des Zuordnens der anhand der verschiedenen Spektralsignale bestimmten optischen Verzögerung oder Verzögerungen zu einer oder mehreren sogenannten Grenzflächenkurven (521, 522, 523, 524), die Geraden mit einheitlicher positiver oder negativer Steigung oder Null-Steigung entsprechen, in Abhängigkeit von der jeweiligen optischen Verzögerung der Erfassung der Spektralsignale; und
- des Ableitens hieraus von Informationen über die Struktur und/oder die Lage von Grenzflächen des Objekts (13) unter Verwendung der Grenzflächenkurve oder -kurven (521, 522, 523, 524).

10. Verfahren nach Anspruch 9, welches das Erfassen einer Vielzahl von Spektralsignalen für eine Vielzahl von optischen Verzögerungen in einem Bereich von optischen Verzögerungen umfasst, die das Erzeugen von optischen Weggleichheiten zwischen dem optischen Referenzstrahl und dem optischen Messstrahl ermöglichen, wenn sich das Objekt (13) in einem sogenannten zeitlichen Messbereich befindet.

11. Verfahren nach einem der Ansprüche 9 oder 10, welches das Erfassen einer Vielzahl von Spektralsignalen für eine Vielzahl von optischen Verzögerungen umfasst, die um ein Inkrement beabstandet sind, das höchstens der Hälfte des spektralen Messbereichs entspricht.

12. Verfahren nach einem der Ansprüche 9 bis 11, bei dem die Bestimmung von optischen Verzögerungsinformationen zwischen interferierenden Strahlen eine Berechnung eines zeitlichen Interferenzsignals und die Bestimmung von Positionen von Interferenzspitzen umfasst.

13. Verfahren nach einem der Ansprüche 9 bis 12, bei dem die Struktur und/oder die Lage von Grenzflächen des Objekts (13) dadurch bestimmt wird, dass die jeweilige(n) optische Verzögerung(en) ermittelt wird, für die die optische Verzögerung entlang einer Grenzflächenkurve Null ist.

14. Gerät, umfassend eine Vorrichtung nach einem der Ansprüche 1 bis 8.

**Claims**

1. Low-coherence interferometer device (100, 200) for determining information on the structure and/or the location of interfaces of an object (13) comprising:

- a polychromatic light source (11);
- an optical system for generating a measurement optical beam reflected by said object (13), and a reference optical beam;
- a delay line (14) for introducing a variable optical delay between the measurement optical beam and the reference optical beam;
- an optical detection (15) for combining the measurement optical beam and the reference optical beam and producing a spectral signal representative of an optical-power spectral density of the resulting interference signal;
**characterized in that** it also comprises a control and processing module (16) arranged in order to:

- acquire a plurality of spectral signals for a plurality of optical delays;
- determine, for each spectral signal, optical retardation information between interfering beams within a measurement range called spectral measurement range;
- analyze the evolution of said optical retardations depending on the optical delay and assign the optical retardation or retardations determined from the different spectral signals to one or more curves called interface curves (521, 522, 523, 524), corresponding to straight lines having unitary positive or negative gradients or zero gradients, depending on the respective optical delay of the acquisition of said spectral signals; and
- deduce therefrom information on the structure and/or the location of interfaces of the object (13) by using the interface curve or curves (521, 522, 523, 524).

2. The device of claim 1, which comprises a light source (11) emitting a polychromatic light in the form of pulses and an optical detector (15) of the spectral type.

3. The device of claim 1, which comprises a light source (11) with a tuneable or scanning laser, and an intensity optical detector (15).

4. The device of one of claims 1 to 3, which comprises a delay line (14) making it possible to introduce optical delays in discrete steps.

5. The device of claim 4, in which the delay line (14) comprises an optical switch (31).

6. The device of any one of claims 1 or 2, which comprises a delay line (14) making it possible to introduce a continuously variable optical delay.

7. The device of one of the preceding claims, which comprises a common-path interferometer with a measurement arm (21, 22) in order to direct the measurement beam towards the object, and a semi-reflective element inserted in said measurement arm in order to generate the reference optical beam.

8. The device of claim 7, which comprises a differential delay line (240) with an optical reflection (29) at a fixed position, and the delay line (14).

9. Method for determining information on the structure and/or the location of interfaces of an object utilizing a low-coherence interferometer (100, 200), comprising the steps of:

  - emitting a polychromatic light with a polychromatic light source (11);
  - generating a measurement optical beam reflected by said object (13) to be measured, and a reference optical beam;
  - introducing a variable optical delay between the measurement optical beam and the reference optical beam with a delay line (14);
  - combining, by means of an optical detection (15), the measurement optical beam and the reference optical beam and producing a spectral signal representative of an optical-power spectral density of the resulting interference signal;
  - acquiring a plurality of spectral signals for a plurality of optical delays;
  - determining, for each spectral signal, optical retardation information between interfering beams within a measurement range called spectral measurement range;
  - analyzing the evolution of said optical retardations depending on the optical delay;
  **characterized in that** it also comprises the steps of:

  - assignment of the optical retardation or retardations determined from the different spectral signals to one or more curves, called interface curves (521, 522, 523, 524), corresponding to straight lines having unitary positive or negative gradients or zero gradients, depending on the respective optical delay of acquisition of said spectral signals; and
  - deducing information on the structure and/or the location of interfaces of the object (13) by using the interface curve or curves (521, 522, 523, 524).

10. The method of claim 9, which comprises acquiring a plurality of spectral signals for a plurality of optical delays within a range of optical delays making it possible to generate optical route equalities between the reference optical beam and the measurement optical beam when the object (13) is located within a measurement range called temporal measurement range.

11. The method of any one of claims 9 or 10, which comprises acquiring a plurality of spectral signals for a plurality of optical delays spaced apart by an increment corresponding at most to half of the spectral measurement range.

12. The method of one of claims 9 to 11, in which determining optical retardation information between interfering beams comprises calculating a temporal interference signal, and determining positions of interference peaks.

13. The method of one of claims 9 to 12, in which the structure and/or location of interfaces of the object (13) is determined by determining the respective optical delay or delays for which the optical retardation along an interface curve is zero.

14. Appliance comprising a device according to any one of claims 1 to 8.

**Fig. 1**

**Fig. 2**

**Fig. 3**

**Fig. 4**

**Fig. 5**

**Fig. 6**

**EP 3 601 946 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7426036 B
- US 2012257207 A1